# EUROPÄISCHE PATENTSCHRIFT

(11) **EP 3 829 677 B1**
(45) Veröffentlichungstag und Bekanntmachung des Hinweises auf die Patenterteilung: **02.09.2026**
(21) Anmeldenummer: 19746464.7
(22) Anmeldetag: 26.07.2019
(51) Int. Cl.: A61M 5/14, A61M 5/142, A61M 5/145, A61M 5/168

(54) **INFUSIONSSYSTEM MIT INFUSIONSPUMPE UND DAMIT KUPPELBAREN PUMPMODUL**
INFUSION SYSTEM WITH INFUSION PUMP AND DETACHABLE PUMP MODULE
SYSTÈME DE PERFUSION AVEC POMPE À PERFUSION ET MODULE DE POMPE DÉTACHABLE

(30) Priorität: 01.08.2018 DE 102018118630
(43) Veröffentlichungstag der Anmeldung: 09.06.2021
(73) Patentinhaber: B. Braun Melsungen AG, 34212 Melsungen (DE)
(72) Erfinder: FUCHS, Jürgen, 34308 Bad Emstal (DE); HARTUNG, Jürgen, 34298 Helsa (DE); STEGER, Jürgen, 34327 Körle (DE)
(74) Vertreter: Winter, Brandl - Partnerschaft mbB
(86) Internationale Anmeldenummer: PCT/EP2019/070214
(87) Internationale Veröffentlichungsnummer: WO 2020/025484

(56) Entgegenhaltungen:
- EP-A2- 0 110 276
- WO-A1-2016/176198
- US-A1- 2002 176 788
- US-A1- 2011 150 680

## Beschreibung

Die vorliegende Erfindung betrifft ein Infusionssystem mit einer Infusionspumpe und einem austauschbaren, separaten Pumpmodul, wobei das Pumpmodul in einer Aufnahme der Infusionspumpe anordbar oder angeordnet ist und eine mit einem Antriebsmechanismus der Infusionspumpe wirkverbindbare Kolben-Zylinder-Einheit mit einem in einem Zylinder translatorisch bewegbaren Kolben umfasst/aufweist, wobei der Kolben mittels einer mit dem Kolben wirkverbundenen Koppel- oder Kuppelstruktur und einer zum verbindenden Eingreifen mit der Kuppelstruktur ausgebildeten und mit dem Antriebsmechanismus wirkverbundenen Gegenkoppel- oder Gegenkuppelstruktur mit dem Antriebsmechanismus kuppelbar ist.

### Allgemeiner technischer Hintergrund

In der Infusionstechnik sind allgemein Schlauchpumpen sowie Spritzenpumpen bekannt, um einem Patienten Flüssigkeit zuzuführen. Für Anwendungen, die im Hinblick auf die Dosiergenauigkeit hohe Anforderungen stellen, werden in der Regel Spritzenpumpen verwendet, da sich mit diesen eine definierte Fluidförderung gut und exakt erzielen lässt. Bei Anwendungen, die ein größeres Fördervolumen an Fluid verlangen, sind Schlauchpumpen gut geeignet, da sie in Folge ihrer Fördertechnik weitgehend kontinuierlich und ohne diskretes Fördervolumen fördern.

### Stand der Technik

Aus dem Stand der Technik sind solche Anwendungsfälle einer Infusionspumpe bekannt, in denen relativ hohe Fördervolumina gepaart werden müssen mit einer hohen Fördergenauigkeit, was zu großen Problemen führt. Spritzenpumpen weisen nämlich den Nachteil auf, dass das mit ihnen förderbare Fluidvolumen begrenzt ist, nämlich durch das Volumen der verwendeten Spritze (Zylindervolumen). Schlauchpumpen hingegen können zwar relativ große Fördervolumina schaffen, besitzen jedoch den Nachteil einer gegenüber der Spritzenpumpe geringen Fördergenauigkeit. Insbesondere bei kleinen Förderraten ist ihre Fördergenauigkeit auf Grund der üblicherweise eingesetzten Peristaltik des Schlauchs nicht gewährleistet. Außerdem ist der Antrieb einer Schlauchpumpe in der Regel relativ groß und schwer und verbraucht durch das Walken des Pumpenschlauchs durch den Peristaltikantrieb verhältnismäßig viel Energie.

Der vorstehenden Problematik begegnet ein Infusionssystem des Anmelders, bei dem ein Pumpmodul mit einer Kolben-Zylinder-Einheit vorgesehen ist, die dafür angepasst ist, mittels eines Antriebsmechanismus der Infusionspumpe betätigt zu werden, wobei eine Ventileinrichtung vorgesehen ist, um im Fall eines Pumpförderhubs eine Verbindung zwischen der Kolben-Zylinder-Einheit als Flüssigkeit-Zwischenspeicher und einem Versorgungsanschluss zu einem separaten Flüssigkeitsgroßvolumen zu unterbrechen sowie eine Verbindung zwischen der Kolben-Zylinder-Einheit und einem Patientenanschluss freizugeben und im Fall eines Pumpsaughubs die Verbindung zwischen der Kolben-Zylinder-Einheit und dem Versorgungsanschluss zu dem separaten Flüssigkeitsgroßvolumen freizugeben sowie die Verbindung zwischen der Kolben-Zylinder-Einheit und dem Patientenanschluss zu sperren. Das als Einmalartikel konzipierte, separat zur Infusionspumpe ausgebildete Pumpmodul wird austauschbar in der Multi-Use-Infusionspumpe angeordnet, was insbesondere für Home-Infusionssysteme unter anderem den großen Vorteil beinhaltet, dass die Sterilität bei einer Infusion einfach sicherzustellen ist. Jedoch sind weitere wichtige Aspekte bei der Verabreichung einer Infusion und bei der Verwendung des Infusionssystems zu beachten, wie zum Beispiel dass Fehlfunktionen und Fehlbedienungen des Systems möglichst komplett ausgeschlossen werden können und dass ein bestimmungsgemäßer Ablauf einer Behandlung garantiert werden kann.

US 2011/150680 A1 offenbart ein Infusionssystem mit einer Infusionspumpe und einem hierzu separaten, als Einwegprodukt vorgesehenen und damit austauschbaren Pumpmodul, das in einer Aufnahme der Infusionspumpe anzuordnen ist, wobei das Pumpmodul einen Pumpkolben besitzt, der mit Hakenelementen mit einer Schubantriebsstange der Infusionspumpe koppelbar ist.

US 2002/176788 A1 offenbart ein Infusionssystem mit einer Infusionspumpe und einem austauschbaren Pumpmodul, das über eine Koppelstruktur mit einer Gegenkoppelstruktur der Infusionspumpe gekoppelt und durch ein Sicherungselement verriegelt.

Relevanter Stand der Technik ist ferner in WO 2016/176198 A1 und EP 0 110 276 A2 offenbart.

### Kurzbeschreibung der Erfindung

Ausgehend von der vorstehend erläuterten Problematik liegt der Erfindung die Aufgabe zugrunde, die zuvor angeführten Nachteile zu beseitigen, insbesondere ein Infusionssystem bereit zu stellen, das ein besonders einfaches, anwenderfreundliches und sicheres Koppeln/Kuppeln des Pumpmoduls mit der Infusionspumpe ermöglicht und sicherstellt, dass das System erst in Betrieb genommen werden kann, wenn das Pumpmodul in bestimmungsgemäßer Weise (d.h. korrekt in die Aufnahme auf Seiten der Infusionspumpe eingeführt) mit der Infusionspumpe gekuppelt ist, und dass bevorzugt das System nicht ohne weiteres während einer laufenden Behandlung außer Betrieb genommen werden kann.

Diese Aufgabe wird nach der Erfindung gelöst durch ein Infusionssystem mit den Merkmalen des Patentanspruchs 1. Vorteilhafte Ausgestaltungen der Erfindung sind Gegenstand der Unteransprüche.

Ein erster Aspekt betrifft ein Infusionssystem mit einer Infusionspumpe und einem hierzu separaten, austauschbaren Pumpmodul, wobei das Pumpmodul in einer Aufnahme der Infusionspumpe anordbar/angeordnet ist und eine mit einem Antriebsmechanismus der Infusionspumpe wirkverbindbare Kolben-Zylinder-Einheit mit einem in einem Zylinder translatorisch bewegbaren Kolben umfasst, wobei der Kolben mittels einer mit dem Kolben wirkverbundenen Koppel-/Kuppelstruktur und einer zum verbindenden Eingreifen mit der Kuppelstruktur ausgebildeten und mit dem Antriebsmechanismus wirkverbundenen Gegenkoppel-/Gegenkuppelstruktur mit dem Antriebsmechanismus kuppelbar ist, wobei das Infusionssystem ein Sicherungselement aufweist, das in einer Sicherungsstellung die Kuppelstruktur und die Gegenkuppelstruktur im gekuppelten Zustand miteinander verriegelt. Der erste Aspekt alleine ist nicht durch die beanspruchte Erfindung abgedeckt.

Die Erfindung betrifft in einem zweiten Aspekt (der. ggf. mit dem ersten Aspekt kombinierbar ist) ein Infusionssystem mit einer Infusionspumpe und einem austauschbaren Pumpmodul, wobei das Pumpmodul in einer Aufnahme der Infusionspumpe anordbar ist und eine mit einem Antriebsmechanismus der Infusionspumpe wirkverbindbare Kolben-Zylinder-Einheit mit einem in einem Zylinder translatorisch bewegbaren Kolben umfasst, wobei der Kolben mittels einer mit dem Kolben wirkverbundenen Kuppelstruktur und einer zum verbindenden Eingreifen mit der Kuppelstruktur ausgebildeten und mit dem Antriebsmechanismus wirkverbundenen Gegenkuppelstruktur mit dem Antriebsmechanismus kuppelbar ist, wobei die Infusionspumpe eine Steuerkulisse aufweist, die derart mit dem Pumpmodul wirkverbunden ist, dass sie bei bestimmungsgemäß in der Aufnahme angeordnetem Pumpmodul in eine Freigabestellung oder Pumpstellung oder Infusionsstellung zwangspositioniert ist, in der eine Pumpaktion des Kolbens möglich ist, und dass sie bei nicht bestimmungsgemäß in der Aufnahme angeordnetem Pumpmodul in eine Sperrstellung oder Entkopplungsstellung zwangspositioniert ist, in der eine Pumpaktion des Kolbens nicht möglich ist.

In einem dritten Aspekt (der. ggf. mit dem ersten und/oder zweiten Aspekt kombinierbar ist) betrifft die Erfindung ein Infusionssystem mit einer Infusionspumpe und einem austauschbaren Pumpmodul, wobei das Pumpmodul in einer Aufnahme der Infusionspumpe anordbar ist und eine mit einem Antriebsmechanismus der Infusionspumpe wirkverbindbare Kolben-Zylinder-Einheit mit einem in einem Zylinder translatorisch bewegbaren Kolben umfasst, wobei der Kolben mittels einer mit dem Kolben wirkverbundenen Kuppelstruktur und einer zum verbindenden Eingreifen mit der Kuppelstruktur ausgebildeten und mit dem Antriebsmechanismus wirkverbundenen Gegenkuppelstruktur mit dem Antriebsmechanismus kuppelbar ist, wobei das Infusionssystem ein Sicherungselement aufweist, das in einer Sicherungsstellung die Kuppelstruktur und die Gegenkuppelstruktur im gekuppelten Zustand miteinander verriegelt und wobei die Infusionspumpe eine Steuerkulisse aufweist, die derart mit dem Pumpmodul wirkverbunden ist, dass sie bei bestimmungsgemäß in der Aufnahme angeordnetem Pumpmodul in eine Freigabestellung zwangspositioniert ist, wobei das Infusionssystem ein Sicherungselement aufweist, das in einer Sicherungsstellung die Kuppelstruktur und die Gegenkuppelstruktur im gekuppelten Zustand miteinander verriegelt.

Vorteilhafte Ausführungsformen der Erfindung sind in den Unteransprüchen beansprucht und werden nachfolgend näher erläutert.

Eine bevorzugte Ausführungsform des Infusionssystems ist dadurch gekennzeichnet, dass die Infusionspumpe ein Deckelelement aufweist. Dieses kann insbesondere in eine die Aufnahme für das Pumpmodul verschließende Schließstellung (und in eine die Aufnahme freigebende Offenstellung) positionierbar sein. Das Deckelelement, die Aufnahme und das Pumpmodul sind vorzugsweise derart aufeinander abgestimmt und ausgebildet, dass das Deckelelement bei nicht bestimmungsgemäß in der Aufnahme angeordnetem Pumpelement nicht in die die Aufnahme verschließende Schließstellung positionierbar ist. Auf diese Weise wird dem Anwender vor Inbetriebnahme der Pumpe eine Rückmeldung darüber gegeben, ob das Pumpmodul korrekt in der Infusionspumpe angeordnet und mit dieser verbunden ist oder nicht.

Bei einer weiteren bevorzugten Ausführungsform ist das Deckelelement mit der Steuerkulisse wirkverbunden. Die Wirkverbindung ist vorzugsweise derart beschaffen, dass diese bei in der Schließstellung befindlichem Deckelelement in die Freigabestellung zwangspositioniert ist, und bei nicht in der Schließstellung befindlichem Deckelelement in die Sperrstellung zwangspositioniert ist. Die Wirkverbindung kann insbesondere über einen Mechanismus, insbesondere einen Kniehebelmechanismus realisiert sein. Er kann insbesondere einen ersten Hebelarm und einen zweiten Hebelarm aufweisen, die miteinander über ein freies Gelenk verbunden sind. Einer der Hebelarme kann im Wesentlichen L-förmig ausgebildet sein. Außerdem kann einer der Hebelarme schwenkbar an der Infusionspumpe, insbesondere mittels eines an deren Gehäuse angeordneten fixen Drehgelenks, angeordnet sein. Der Kniehebelmechanismus kann außerdem mit einer mit dem Deckelelement zusammenwirkenden Betätigungsstange (Nocken o.Ä.) gekoppelt sein. Der verbleibende der beiden Hebelarme ist mit seinem dem anderen Hebelarm gegenüberliegenden Ende vorzugsweise drehbar mit der Steuerkulisse gekoppelt.

Nach einer weiteren bevorzugten Ausführungsform ist das Sicherungselement, das insbesondere in Form einer vorzugsweise im Wesentlichen hohlzylinderförmigen Klemmhülse ausgebildet sein kann, mit der Steuerkulisse wirkverbunden. Diese Wirkverbindung ist vorzugsweise derart beschaffen, dass das Sicherungselement die Kuppelstruktur und die Gegenkuppelstruktur bei in der Freigabestellung befindlicher Steuerkulisse fixiert und das Sicherungselement die Kuppelstruktur und die Gegenkuppelstruktur bei in der Sperrstellung befindlicher Steuerkulisse freigibt. Zum Beispiel kann die Steuerkulisse eine Steuernut aufweisen. Vorzugsweise umfasst diese einen in Längsrichtung L verlaufenden Freigabeabschnitt und einen zunächst geneigt und dann quer zur Längsrichtung L verlaufenen Sperrabschnitt. Das Sicherungselement weist in diesem Fall zumindest einen, vorzugsweise zwei einander diametral gegenüberliegende Steuernocken auf, die in die Steuernut der Steuerkulisse eingreifen, so dass das Sicherungselement durch eine Relativpositionierung der Steuerkulisse infolge des vorbeschriebenen Eingriffs zwischen einer Sicherungsstellung oder Klemmstellung und einer Freigabestellung positionierbar ist.

Eine bevorzugte Ausführungsform der Erfindung ist dadurch gekennzeichnet, dass das Infusionssystem, insbesondere die Infusionspumpe, einen Kniehebelmechanismus aufweist. Dieser ist einerseits mit dem Pumpmodul wirkverbunden, insbesondere über eine Ankopplung an das Deckelelement. Andererseits ist er mit der Steuerkulisse wirkverbunden, zum Beispiel indem er an der Steuerkulisse angelenkt ist.

Die Steuerkulisse kann insbesondere in die Sperrstellung / Entkopplungsstellung vorgespannt sein, zum Beispiel mittels eines Federelements wie einer Biegefeder, Druckfeder oder Zugfeder. Auf diese Weise ist sichergestellt, dass eine Pumpaktion bei nicht bestimmungsgemäß angeschlossenem Pumpmodul unmöglich ist.

Nach einer bevorzugten Ausführungsform der Erfindung ist das Sicherungselement als hülsenförmiger Hohlkörper ausgebildet. Die Längsachse des Sicherungselements / Hohlkörpers ist vorzugsweise parallel zur, insbesondere kongruent mit der Kolbenachse ausgerichtet. Außerdem ist das Sicherungselement vorzugsweise relativ zur Kuppelstruktur und Gegenkuppelstruktur in Richtung der Kolbenachse zwischen der Sicherungsstellung und einer Freigabestellung positionierbar.

Das Sicherungselement ist vorzugsweise in die Sicherungsstellung vorgespannt, zum Beispiel mittels eines Federelements wie einer Biegefeder, Druckfeder oder Zugfeder. Auf diese Weise ist sichergestellt, dass das Pumpmodul bei bestimmungsgemäßer Anordnung in der Aufnahme der Infusionspumpe stets gesichert mit deren Antrieb gekoppelt ist und ein versehentliches Lösen sicher vermieden werden kann.

Der Antrieb der Infusionspumpe kann insbesondere eine Antriebsstange und eine Kupplungsstange aufweisen und über diese wirktechnisch mit dem Kolben der Kolben-Zylinder-Einheit kuppelbar sein. Vorzugsweise sind alle diese Elemente in Richtung der Längsachse der Kolben-Zylinder-Einheit angeordnet. Die Antriebsstange und/oder die Kupplungsstange können dauerhaft miteinander und mit dem Antrieb gekoppelt sein. Sie sind also nicht dazu bestimmt, durch einen Anwender von diesem entkoppelt zu werden. Die Antriebsstange ist vorzugsweise translatorisch angetrieben und kann infolgedessen eine Hin-Her-Bewegung in Richtung der Längsachse L ausführen. Die Kupplungsstange kann an ihrem distalen Ende eine Gegenkuppelstruktur aufweisen, die passend zur Kuppelstruktur des Kolbens ausgebildet ist. Die Gegenkuppelstruktur kann insbesondere eine ggf. umlaufende Vertiefung oder Nut aufweisen, die für ein kuppelndes Eingreifen mit Kuppelhaken des Kolbens geeignet und bestimmt ist. Das Sicherungselement ist vorzugweise relativ zur Kupplungsstange in Richtung der Längsachse L zwischen einer distalen Klemmstellung und einer proximalen Freigabestellung positionierbar. In der Klemmstellung übergreift es die miteinander in Eingriff stehende Kuppelstruktur und Gegenkuppelstruktur, so dass sich diese nicht voneinander lösen können und die Verbindung von Kolben/Pumpmodul und Antrieb/Infusionspumpe gesichert ist. In der Freigabestellung gibt das Sicherungselement die Kuppelstruktur und die Gegenkuppelstruktur frei, so dass ein Anwender das Pumpmodul einfach aus der Aufnahme entnehmen kann.

Eine besonders anwenderfreundliche und sichere Ausführungsform des Infusionssystems ist vorzugsweise dadurch gekennzeichnet, dass es eine Notentriegelung mit einem mit dem Sicherungselement gekoppelten Betätigungselement aufweist. Mittels des Betätigungselements kann das Sicherungselement manuell betätigt werden und aus der Sicherungsstellung in eine Freigabestellung gebracht oder überführt werden. Es ist von besonderem Vorteil, wenn das Betätigungselement mit der Steuerkulisse gekoppelt ist, derart, dass es bei in der Freigabestellung befindlicher Steuerkulisse blockiert und nicht zu betätigen ist, um das Sicherungselement aus der Sicherungsstellung in eine Freigabestellung zu bringen. Auf diese Weise kann eine Entkopplung von Pumpmodul und Infusionspumpe während einer Anwendung aufgrund einer versehentlichen Betätigung der Notentriegelung sicher vermieden werden.

Die Aufnahme der Infusionspumpe für das Pumpmodul kann beispielsweise in Form einer Ausnehmung in einem Pumpengehäuse ausgebildet sein und insbesondere mittels eines Deckelelements oder Verschlusses (zum Beispiel in Form einer schwenkbar an der Pumpe, insbesondere an deren Gehäuse, angeordneten Verschlussklappe) zu verschließen sein. Das Deckelelement kann insbesondere in einer die Aufnahme verschließenden Stellung über eine Verriegelungseinheit mit dem Gehäuseteil verriegelbar sein. Der Verschluss, die Aufnahme und das Pumpmodul können derart aufeinander abgestimmt sein, dass ein Schließen (und ggf. Verriegeln) des Verschlusses nur möglich ist, wenn das Pumpmodul in bestimmungsgemäßer Weise und korrekt angeordnet und angeschlossen ist. Derart kann einem Nutzer ein Feedback im Hinblick auf eine fehlerfreie Einrichtung und Nutzung der Pumpe gegeben werden, was in vorteilhafter Weise die Patientensicherheit erhöht.

Außerdem kann durch ein Schließen des Deckelelements ein automatisches Kuppeln der Kolben-Zylinder-Einheit mit dem Antrieb bewirkt werden, was eine besonders einfache und sichere Bedienung darstellt. Ein Gehäuse der Infusionspumpe kann insbesondere aus einem Gehäuseunterteil, an dem die gesamte Mechanik und Elektronik angeordnet und gehalten sein kann, sowie aus einem Gehäuseoberteil, das insbesondere ein Display und diversen Schaltelemente aufweisen kann, bestehen. Im Inneren des Gehäuses kann sich ein Verschlussmechanismus für die Verschlussklappe befinden.

Der Kolben kann insbesondere an einer Kuppelstange, besonders bevorzugt an einem den beiden Schlauchabschnitten zugewandten Ende der Kuppelstange, angeordnet sein. Diese kann mit ihrem anderen Ende aus dem Zylinder herausragen. Der Kolben kann insbesondere länger als der maximale Füllstand des Zylinders sein. Hierdurch wird vermieden, dass sich Umgebungsluft die sich naturgemäß außerhalb des Zylinders befindet mit dem im Zylinder befindlichen Medium vermischt. In der Kombination aus zwei Dichtlippen und Kolben / Zylinderlänge ergibt sich eine wirksame Barriere gegen eindringende Keime. Bevorzugt ist die beidseitige Dichtung des Kolbens der Kolben-Zylinder-Einheit mittels einer proximalen und einer distalen Dichtung realisiert. So ist eine Unterdruckbewegung und eine Überdruckbewegung des Kolbens abgedichtet, um eine Sterilität eines Fluidraums / Dosierraums, das heißt des Raums, in dem die sich die zu fördernde Flüssigkeit im Zylinder aufhält, zu gewährleisten. So erfüllt die Pumpe die hohen Anforderungen hinsichtlich der Sauberkeit in medizinischem Umfeld. Die proximale und die distale Dichtung verlaufen zueinander vorteilhafterweise planparallel. Da der Kolben im Wesentlichen einen runden Querschnitt aufweist, resultieren hieraus etwa ringförmige Dichtungen. Die Distanz zwischen der proximalen und der distalen Dichtung ist insbesondere größer als der Förderhub der Kolben-Zylinder-Einheit ausgestaltet. Hieraus resultiert, dass der Dosierbereich ausschließlich mit dem Kolben und dessen steriler Dichtlippe in Kontakt ist, wodurch eine Verschmutzung / Verunreinigung des Fluidraums / Dosierraums aufgrund zu hoher Hub-Bewegungen konstruktiv unterbunden ist.

Nach einer bevorzugten Ausführungsform weist die Kolben-Zylindereinheit ein Kuppelelement zum lösbaren Kuppeln mit einem entsprechenden Gegenelement eines Antriebs der Infusionspumpe auf. Dieses Kuppelelement kann direkt am Kolben angeordnet oder ausgebildet sein. Das Kuppelelement kann zum Beispiel in Form einer Steckkupplung passend zum Gegenkuppelstruktur ausgebildet sein und beim bestimmungsgemäßen Anordnen des Pumpmoduls in der Infusionspumpe automatisch oder zwangsläufig mit dem Antrieb gekoppelt werden. Vorzugsweise weist die Kupplung Raststrukturen auf, die beide Kuppelelemente aneinander halten und insbesondere ein für einen Anwender hörbares und/oder fühlbares Verrasten der beiden Kuppelelemente ermöglichen.

Bei der Kolben-Zylinder-Einheit handelt es sich vorzugsweise um eine leichtgängige Einheit, um Betätigungskräfte gering zu halten und eine hohe Fördergenauigkeit zu erzielen. Dies kann beispielsweise bewirkt werden, indem der Kolben in Achsrichtung beidseitig mit Dichtungen versehen und derart gegenüber dem Zylinder abgedichtet ist. Insbesondere kann die Kolben-Zylinder-Einheit in Form einer Spritze mit einem leichtgängigen Kolben ausgebildet sein, der Dichtkonturen in Zug- und Druckrichtung aufweist. Hierdurch ist die Verwendung des Kolbens unter Beibehaltung der Dichtwirkung in beide Bewegungsrichtungen möglich.

Der Antriebsmechanismus der Infusionspumpe kann insbesondere aus einem elektrischen Antriebsmotor sowie einem Übersetzungsgetriebe zur Transformation der Rotationsbewegung des Antriebsmotors in eine Translationsbewegung (Hin-Her-Bewegung) des im Zylinder verschiebbar gelagerten Kolbens bestehen. Außerdem kann der Antriebsmechanismus zum Beispiel über eine Steuer-/Regeleinheit der Infusionspumpe gesteuert/geregelt sein.

Die Infusionspumpe kann in einer Ausführungsform eine Ventileinrichtung umfassen, die zum Beispiel an die Steuer-/Regeleinheit angeschlossen und eingerichtet ist, um (ggf. in geregelter/gesteuerter Weise) im Fall eines Pumpförderhubs eine Verbindung zwischen der Kolben-Zylinder-Einheit als Flüssigkeit-Zwischenspeicher und einem Versorgungsanschluss zu einem separaten Flüssigkeitsgroßvolumen zu unterbrechen sowie eine Verbindung zwischen der Kolben-Zylinder-Einheit und einem Patientenanschluss freizugeben und im Fall eines Pumpsaughubs die Verbindung zwischen der Kolben-Zylinder-Einheit und dem Versorgungsanschluss zu dem separaten Flüssigkeitsgroßvolumen freizugeben sowie die Verbindung zwischen der Kolben-Zylinder-Einheit und dem Patientenanschluss zu sperren. Im einfachsten Fall kann die Ventileinrichtung passiv betätigbare Ventilelemente aufweisen, zum Beispiel ein oder zwei Rückschlagventile, die in Verbindungsleitungen, insbesondere in separaten, als Einweg-Artikel ausgebildeten Verbindungsleitungen, zum Patientenanschluss und zum Flüssigkeitsgroßvolumen untergebracht sind, die in die Infusionspumpe einlegbar sind. Dies hat den Vorteil, dass die Ventileinrichtung besonders preisgünstig herstellbar ist und sich damit als Einweg-Artikel zusammen mit dem gesamten Flüssigkeitsleitungssystem entsorgt werden kann.

Alternativ kann die Ventileinrichtung aktiv betätigbaren Ventilelemente, zum Beispiel zwei Schlauchquetschen/Pressen, aufweisen, die auf flexibel deformierbare Abschnitte der beiden Verbindungsleitungen für deren dichtendes Abdrücken einwirken können. Diese Ventilelemente können insbesondere in der Infusionspumpe (oder dem Pumpenmodul) untergebracht sein. Insbesondere kann das ggf. als Einweg-Artikel ausgebildete Flüssigkeitsleitungssystem einen ersten, vorzugsweise elastisch verformbaren Schlauchabschnitt zur Fluidzufuhr (aus dem Flüssigkeitsgroßspeicher in den Zwischenspeicher) und einen zweiten, vorzugsweise elastisch verformbaren Schlauchabschnitt zur Fluidabfuhr (aus dem Zwischenspeicher in Richtung hin zum Patienten) aufweisen, wobei die beiden Schlauchabschnitte für ein Einsetzen in die Infusionspumpe sowie für ein Anschließen an die Kolben-Zylinder-Einheit (Flüssigkeitszwischenspeicher) zum Ansaugen von Fluid aus dem ersten Schlauchabschnitt in den Flüssigkeitszwischenspeicher und zum Ausgeben von Fluid aus dem Flüssigkeitszwischenspeicher zum Patienten ausgebildet sind. Die Schlauchquetschen/Pressen können bevorzugt als Stempel/Stößel oder als Scherenklemmen oder dergleichen mechanische Klemmmittel ausgebildet sein, die beweglich in der Infusionspumpe gelagert sind (sind damit Bestandteil der Infusionspumpe) und von jeweils einem Antrieb betätigt werden können, der an die Steuer-/Regeleinheit angeschlossen ist. Die beiden im Einlass und Auslass befindlichen Schlauchquetschen/Pressen sind vorzugsweise derart mechanisch miteinander gekoppelt, dass mindestens eine Seite den Schlauch sicher abdrückt. Damit beim Wechsel zwischen Einlass und Auslass keine undichte Position entsteht, können die Schlauchquetschen/Pressen vorgespannt sein, so dass diese die ansonsten undichten Positionen überbrücken. Alternativ kann die Steuerzeit der Ventile auch über z.B. Kurvenscheiben oder durch getrennte Ansteuerungen mittels Motoren erfolgen.

Über die Steuereinheit, die zum Beispiel in Form einer motorisch betriebenen Ventilsteuerung realisiert sein kann, kann der Schlauch im Auslauf, also der zweite Schlauchabschnitt, gesperrt, vorzugsweise abgedrückt werden, wenn die Kolben-Zylinder-Einheit aufgezogen wird. Zudem kann der Schlauch im Zulauf, also der erste Schlauchabschnitt, gesperrt, vorzugsweise abgedrückt werden, wenn die Kolben-Zylinder-Einheit ausgedrückt wird. Bezeichnend ist, dass die Kolben-Zylinder-Einheit wie eine Kolbenpumpe angesteuert werden kann. Die Steuereinheit kann insbesondere eine motorisch angetriebene Kipphebeleinheit aufweisen. Diese kann derart ausgebildet sein und wirken, dass sie in einer ersten Kippstellung den ersten Schlauchabschnitt zusammenquetscht und den zweiten Schlauchabschnitt freigibt und in einer zweiten Kippstellung den zweiten Schlauchabschnitt zusammenquetscht und den ersten Schlauchabschnitt freigibt. Die Steuereinheit kann außerdem einen schwenkbar an der Kipphebeleinheit angeordneten Druckstempel aufweisen. Sie kann vor allem einen mit dem ersten Schlauchabschnitt zusammenwirkenden Einlassstempel und/oder einen mit dem zweiten Schlauchabschnitt zusammenwirkenden Auslassstempel umfassen. Zumindest ein Druckstempel kann mittels einer Vorspanneinheit, insbesondere mittels einer Druckfeder, in eine den jeweiligen Schlauchabschnitt freigebenden, insbesondere nicht kontaktierende, Position vorgespannt sein. Auf diese Weise kann bewirkt werden, dass sich die Druckstempel nicht im Bereich des entsprechenden Schlauchabschnitts befinden, wenn die Pumpe nicht in Betrieb ist. Dies ermöglicht ein besonders anwenderfreundliches Wechseln, Einsetzen und Entfernen des erfindungsgemäßen Pumpmoduls.

Eine bevorzugte Ausführungsform des Pumpmoduls sieht vor, dass der erste Schlauchabschnitt an dem der Kolben-Zylinder-Einheit gegenüberliegenden Ende mit einem Luer-Lock-Kupplungsstück versehen ist. Dieses kann insbesondere als Luer-Lock-Innenkegel ausgebildet sein. Alternativ oder zusätzlich kann der zweite Schlauchabschnitt an dem der Kolben-Zylinder-Einheit gegenüberliegenden Ende mit einem Luer-Lock-Kupplungsstück versehen sein. Dieses kann insbesondere als Luer-Lock-Außenkegel ausgebildet sein. Dies ermöglicht eine besonders einfache zu betätigende Möglichkeit zur Verbindung des Pumpmoduls bzw. dem darin aufgenommenen Flüssigkeitsleitungssystem mit einer extrakorporalen Leitung oder Leitungssystem. Die Erfindung lässt sich damit ohne weiteres und in bekannter Weise zusammen mit bestehenden und weitverbreiteten medizintechnischen Einrichtungen verwenden. Alternativ zu einem Luer-Lock-Anschluss kann eine am Patienten angeschlossene Tropfkammer vorgesehen sein oder der zweite Schlauchabschnitt ist als Sackleitung mit einem verschlossen Ende ausgebildet und kann mit einem Einsteckdorn angeschlossen werden. Zusätzlich oder alternativ bietet sich auch das Anordnen eines Spikes / Einstechdorns an, um die Flexibilität im Anwendungsgebiet bzw. die Kompaktheit der Pumpe zu erhöhen.

Die Steuereinheit und der Antrieb können insbesondere an oder in einem Gehäuseteil der Infusionspumpe angeordnet sein.

Das Pumpmodul kann insbesondere anwenderseitig austauschbar an dem Gehäuseteil anzuordnen sein. Dabei ist es vorzugsweise ohne Nutzung besonderer Werkzeuge oder Vorrichtungen in der Pumpe anzuordnen und/oder aus dieser zu entfernen. Das erfindungsgemäße Pumpmodul kann insbesondere als Einmalartikel ausgebildet und für eine einzige Verwendung in der Pumpe bestimmt sein. Dies ist im Hinblick auf Sterilbedingungen besonders vorteilhaft und anwenderfreundlich. Es kann zum Beispiel in Form einer Einmalspritze realisiert sein, mit deren Fördervolumenöffnung die beiden Schlauchabschnitte strömungstechnisch verbunden sind. Dies kann derart realisiert sein, dass die beiden Schlauchabschnitt eine fortlaufende Fluidleitung ausbilden, die in einem Bereich zwischen den Deformationsstellen mit einer Abzweigung zum Anschließen der Kolben-Zylinder-Einheit, insbesondere einer Einmalspritze, versehen ist. Alternativ kann die Kolben-Zylinder-Einheit / die Einmalspritze zwei Strömungsöffnungen aufweisen, einen Ausgang und einen Eingang, die jeweils strömungstechnisch mit dem entsprechenden Schlauchabschnitt verbunden werden.

Es ist von besonderem Vorteil, wenn der Antrieb der Kolben-Zylinder-Einheit ein Linearantrieb/-motor ist, der in der Bewegungsachse des Kolbens angeordnet ist. Besonders bevorzugt ist, wenn der Antrieb derart angeordnet und ausgebildet ist, dass vom Antrieb auf den Kolben wirkende Kräfte zentral und in Achsrichtung eingeleitet werden. Dabei ist von besonderem Vorteil, dass auf den Kolben wirkende Kräfte minimiert werden können (im Vergleich zu einer dezentralen Krafteinleitung) und derart nur eine geringe Energie zur Betätigung der Kolben-Zylinder-Einheit erforderlich ist. In der Folge kann der Antrieb und damit die Pumpe klein ausgebildet sein, was zu Einsparungen hinsichtlich Gewicht, Kosten und Bauraum führt. Außerdem kann durch die zentrale Einleitung von Betätigungskräften in den Kolben eine hohe Präzision erzielt werden. Die Verwendung einer kleinen Spritze als Kolben-Zylinder-Einheit ermöglicht eine Anordnung eines Linearantriebs unmittelbar in der Achse des Spritzenkolbens, wodurch erreicht wird, dass keine die Genauigkeit beeinflussenden Querkräfte auf den Spritzenkolben wirken. Des Weiteren erzeugt ein kleiner Querschnitt der Spritze nur geringe Kräfte, was eine Verwendung eines einfachen kostengünstigen Antriebs, zum Beispiel eines Linear-Schrittmotors, ermöglicht. Die Baugröße der Pumpe kann dadurch deutlich geringer ausfallen als bei bekannten Infusionspumpen.

Die Infusionspumpe kann außerdem einen zweiten Antrieb, insbesondere Linearantrieb/-motor, für die Steuereinheit aufweisen. Die beiden Antriebe für die Kolben-Zylinder-Einheit und die Steuereinheit sind vorzugsweise derart steuerungstechnisch miteinander gekoppelt, dass die vorstehend beschriebene Steuerungsfunktion durch Abquetschen der beiden Schlauchabschnitte erfolgt.

Da der Antrieb / die Antriebe der Pumpe klein ausgelegt sein können, ist es in vorteilhafter Weise möglich, dass die Pumpe eine Energiespeichereinheit, insbesondere einen Akku, umfasst und ohne direkten Netzanschluss betrieben werden kann. Die Energiespeichereinheit kann zum Beispiel ein üblicher Lithium-Akku sein, der über eine USB-Schnittstelle geladen werden kann. Diese kann außerdem genutzt werden, um Daten in eine Pumpensteuerung ein- und/oder auszulesen.

Das Pumpmodul kann eine Schiebeklemme aufweisen, die derart einstellbar ist, dass sie einen Fluidfluss in dem ersten und/oder dem zweiten Schlauchabschnitt wahlweise freigibt oder sperrt. So ist die Schiebeklemme derart einstellbar, dass sich nach der Inbetriebnahme des Pumpmoduls keine Luftblasen bilden. Die Schiebklemme ist vorzugsweise auf einen Griffabschnitt des Pumpmoduls aufclipsbar, sodass diese leicht aus einem anderen Material herstellbar ist als das restliche Pumpmodul. An einem der Schiebeklemme abgewandten Ende des Pumpmoduls bzw. des Griffabschnitts kann eine Hülse / ein Ring angeordnet sein. Diese Hülse ist geeignet, den ersten und/oder den zweiten Schlauchabschnitt zu fixieren und somit eine sichere Kopplung einer Infusionsleitung mit dem Pumpmodul zu ermöglichen. Sie dient demnach als Haltering, durch den die Infusionsleitung einfädelbar ist.

Zusammenfassend kann man sagen, dass die Erfindung ein Infusionssystem mit der Genauigkeit einer Spritzenpumpe und dem Fördervolumen oder Volumenvorrat einer Schlauchpumpe bei deutlich geringerem Energiebedarf, reduzierter Baugröße und geringen Herstellungskosten bei hoher Anwenderfreundlichkeit und Sicherheit ermöglicht. Durch die Erfindung können insbesondere die folgenden Vorteile erzielt werden:
- Einfachere Bedienung für den Anwender
- Das System ermöglicht den Betrieb der Pumpe nur bei richtiger Anwendung

Weitere Merkmale und Vorteile der Erfindung ergeben sich aus der folgenden beispielhaften und nicht beschränkenden Beschreibung der Figuren. Diese sind lediglich schematischer Natur und dienen nur dem Verständnis der Erfindung. Dabei zeigen:
Fig. 1 eine perspektivische Ansicht der Rückseite eines Infusionssystems mit einer Infusionspumpe und einem darin eingesetzten Pumpmodul,
Fig. 2 das Infusionssystem der Figur 1 in einer Schnittdarstellung bei teilweise freigeschnittenem Pumpengehäuse in einer Koppelstellung oder Infusionsstellung,
Fig. 3 eine Schnittdarstellung der Infusionspumpe der Figuren 1 und 2 mit darin eingesetztem Pumpmodul in einer Entkopplungsstellung,
Fig. 4 eine Schnittdarstellung quer zur Schnittebene der Figuren 2 und 3 der Infusionspumpe mit darin eingesetzten Pumpmodul,
Fig. 5 eine weitere Schnittdarstellung quer zur Schnittebene der Figuren 2 und 3 der Infusionspumpe mit darin eingesetzten Pumpmodul während einer Betätigung einer Notentriegelung,
Fig. 6 eine Ausführungsform eines Pumpmoduls mit daran angeschlossener Schiebeklemme zum Abklemmen einer der Fluidleitungen und
Fig. 7 eine perspektivische Ansicht der Vorderseite eines Infusionssystems mit einer Infusionspumpe und einem darin eingesetzten Pumpmodul.

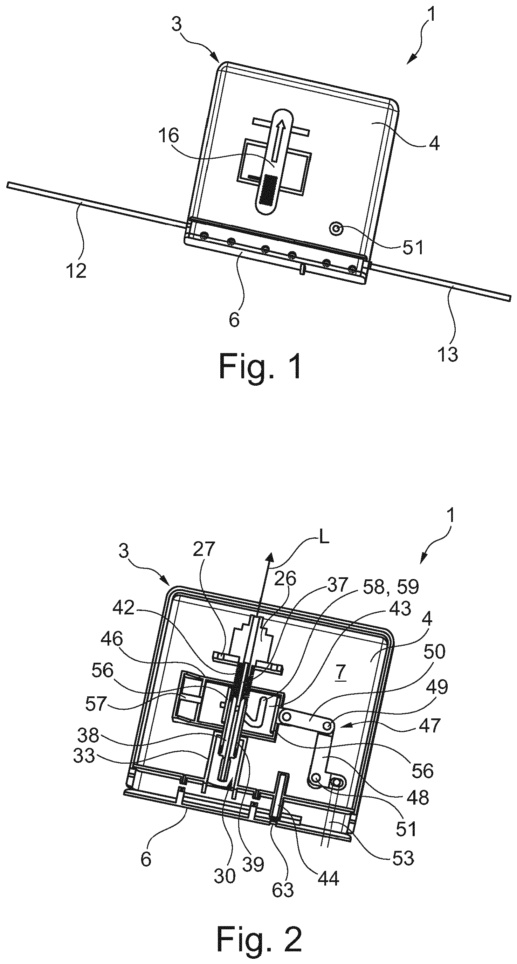

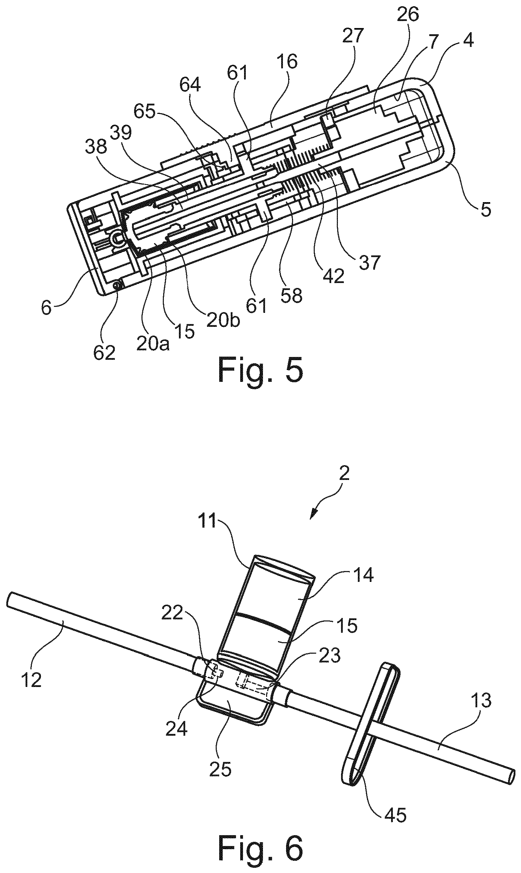

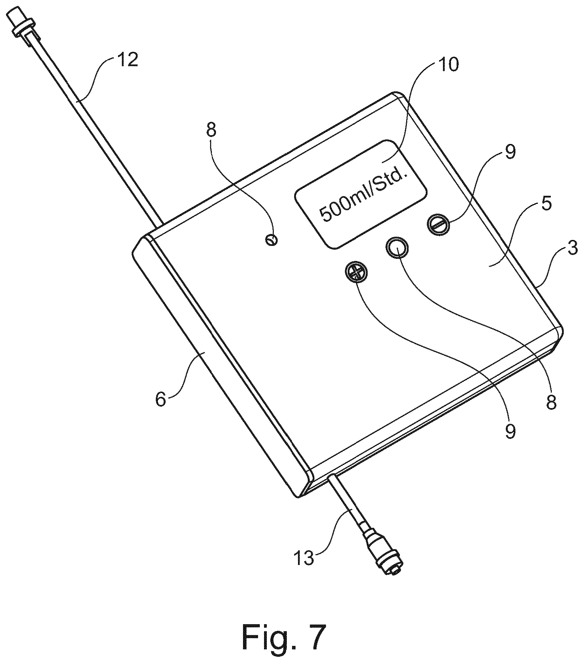

Die Figuren 1 und 7 zeigen ein Ausführungsbeispiel eines Infusionssystems nach der Erfindung mit einer Infusionspumpe 1 und mit einem darin eingesetzten Pumpmodul 2 (Figur 1 Rückseite, Figur 7 Vorderseite). Die Pumpe 1 weist ein Gehäuse 3 auf, das im Wesentlichen bevorzugt aus einem Gehäuseunterteil 4 mit einem Gehäusedeckel 5 und einem Deckelelement 6 hier in Form einer Verschlussklappe 6 besteht. Das Gehäuseunterteil 4 und der Gehäusedeckel 5 sind miteinander verbindbar/verbunden, zum Beispiel über in den Figuren nicht dargestellte Rastverbindung oder Verschraubungen, und bilden im zusammengefügten Zustand das Gehäuse 3 aus. Das Gehäuseunterteil 4 weist einen Gehäuseboden 7 auf. Im oder am Gehäusedeckel 5 sind Anzeigeelemente 8, Bedienungselemente 9 und ein Anzeigedisplay 10 der Pumpe 1 angeordnet. Im Gehäuseunterteil 4 ist ein Betätigungselement 16 hier in Form eines Schieberiegels 16 angeordnet. In dem Gehäuse 3 ist eine Aufnahme 30 für das Pumpmodul 2 ausgebildet (siehe insbesondere in Figur 2), die mittels des Deckelelements 6 verschließbar ist. Die Aufnahme 30 ist in distaler Richtung offen, in proximaler Richtung mittels einer Bodenwand 32 geschlossen und von einer der Außenform der Kolben-Zylinder-Einheit entsprechenden Seitenwand 33 (Seitenwänden 33) umschlossen. Insgesamt ist die Aufnahme 30 passend zur Kolben-Zylinder-Einheit 11 (dargestellt in Figur 6) ausgebildet, so dass diese darin lagebestimmt angeordnet werden kann. In der Bodenwand 32 der Aufnahme 30 ist eine zentrale Durchgangsöffnung ausgebildet, durch die wie später beschrieben ist, eine Kuppelstange 38 und eine Klemmhülse 39 als Sicherungselement 39 zum Kuppeln des Kolbens 15 mit dem Antrieb 26 hindurchgeführt sind.

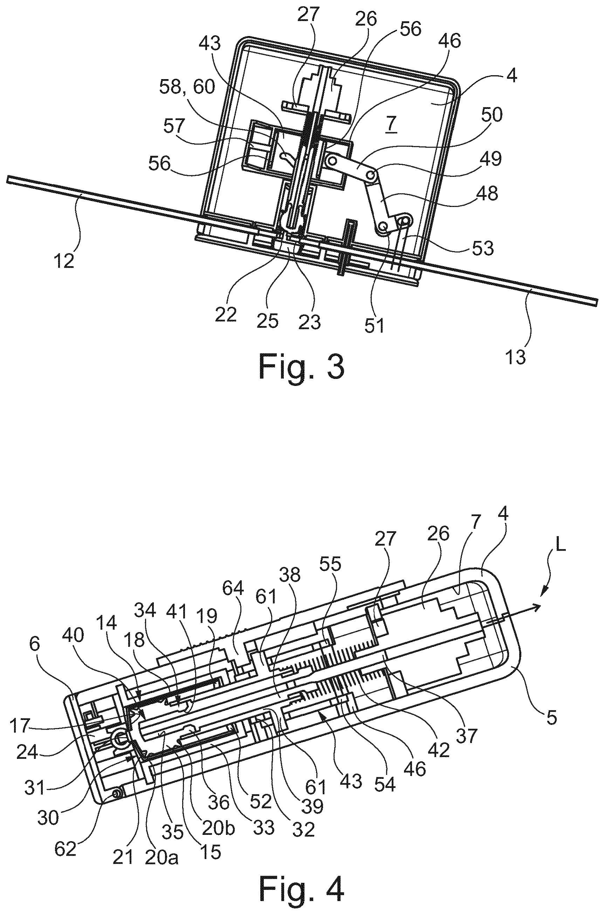

Das Pumpmodul 2 ist einzeln schematisch in Figur 6 gezeigt. Eine detailliertere Darstellung des Pumpmoduls 2 findet sich in den Figuren 3, 4 und 5. Es weist eine Kolben-Zylinder-Einheit 11, einen ersten elastisch verformbaren Schlauchabschnitt 12 und einen zweiten elastisch verformbaren Schlauchabschnitt 13 auf. Das Pumpmodul 2 ist als Einmalartikel (Single Use Artikel, Wegwerfartikel) ausgebildet. Die Schlauchabschnitte 12, 13 können insbesondere als PVC-Schlauch ausgebildet sein.

Die Kolben-Zylinder-Einheit 11 hat einen Zylinder 14, der insbesondere aus Kunststoff bestehen und/oder als Spritzgussteil hergestellt sein kann, und einen darin reziprok hin-und-her bewegbaren Kolben 15, der ebenfalls insbesondere aus Kunststoff bestehen und/oder als Spritzgussteil hergestellt sein kann. Der Zylinder 14 weist eine Bodenwand 17, eine Umfangswand 18 und eine Deckelwand 19 auf. Der Kolben 15 ist mittels beiderseits daran angeordneten Dichtungen 20a, 20b gegenüber der Umfangswand 18 abgedichtet, so dass die Kolben-Zylinder-Einheit 11 einen Fluidraum / Dosierraum 21 aufweist. Der Kolben 15 weist auf seiner proximalen Seite, d.h. auf der dem Fluidraum 21 gegenüberliegenden Seite, eine Kuppelstruktur 34 auf. Diese ist gebildet durch ein zentrales in den Kolben 15 eingebrachtes Sackloch 35, an dessen proximalem Rand mit Rastkonturen versehene Kuppelhaken 36 angeordnet sind. In der Bodenwand 17 ist ein Fluidkanal 31 ausgebildet. An ihrem distalen Endbereich weist die Kolben-Zylinder-Einheit 11 einen Zylinderkopf 24 auf, an dem wiederum distal ein Griffelement 25 angeordnet ist, zur anwenderseitigen Handhabung des Pumpmoduls 2 insbesondere beim Einsetzen in oder beim Herausnehmen aus der Aufnahme 30 der Infusionspumpe 1. Ein in der Seitenwand 18 befindlicher Ringwulst 52 stellt einen AxialAnschlag für den Kolben 15 dar, so dass der maximale Hub der Kolben-Zylinder-Einheit 11 festgelegt ist und ein Herausfallen des Kolbens 15 aus dem Zylinder 14 formschlüssig vermieden wird.

Der Kolben 15 ist vorzugsweise als Leichtlaufkolben angeordnet/ausgebildet/fixiert und weist an seinem distalen und an seinem proximalen Ende eine als bewegliche Dichtlippe ausgestaltete Dichtung 20a, 20b auf. Die Distanz entlang der Längsrichtung L zwischen der distalen Dichtung 20a und der proximalen Dichtung 20b des Kolbens 15 übersteigt in dem gezeigten Ausführungsbeispiel den Kolbenhub der Kolbenzylindereinheit 11. Somit ist garantiert, dass die Zylinderwandung des Fluidraums / Dosierraums 21 ausschließlich mit der einen, distalen Dichtung 20a in Kontakt ist, wodurch dessen Sterilität sichergestellt ist. Vorzugsweise beträgt der Überschuss zwischen der Distanz entlang der Längsrichtung L zwischen der distalen Dichtung 20a und der proximalen Dichtung 20b des Kolbens 15 und dem Kolbenhub mindestens 2 mm.

In dem Zylinderkopf 24 sind eine Einlaufbohrung 22 und eine Auslaufbohrung 23 ausgebildet (siehe Figur 6), die einerseits strömungstechnisch mit dem Fluidkanal 31 und andererseits strömungstechnisch mit dem ersten Schlauchabschnitt 12 und dem zweiten Schlauchabschnitt 13 verbunden sind. Auf diese Weise ist der Fluidraum 21 über den Fluidkanal 31 und die Einlaufbohrung 22 strömungstechnisch mit dem ersten Schlauchabschnitt 12 und über den Fluidkanal 31 und die Auslaufbohrung 23 strömungstechnisch mit dem zweiten Schlauchabschnitt 13 verbunden. Die Schlauchabschnitte 12, 13 können insbesondere an den Zylinder 14 bzw. den Zylinderkopf 24 angeformt sein. Sie können insbesondere wie übliche Infusionsschläuche ausgebildet sein. Der erste Schlauchabschnitt 12 ist in der Ausführungsform aus Figur 3 an seinem von der Kolben-Zylinder-Einheit 11 abgewandten Ende mit einem Einlass-Luer-Lock-Anschluss (nicht dargestellt) versehen. Der zweite Schlauchabschnitt 13 ist an seinem von der Kolben-Zylinder-Einheit 11 abgewandten Ende mit einem Auslass-Luer-Lock-Anschluss (nicht dargestellt) versehen. Damit ist das Pumpmodul 2 einfach an übliche Infusionseinheiten anzuschließen.

Die Figuren 2 bis 5 zeigen, dass die Pumpe 1 einen (pumpeneigenen) Antrieb 26 für die Kolben-Zylinder-Einheit 11 aufweist, hier in Form eines Linearmotors 26. Der Motor 26 ist kongruent zur Längsachse L der Kolben-Zylinder-Einheit 11 angeordnet, so dass Kräfte zum reziproken Antreiben des Kolbens 15 zentral und ohne Verwindungen / Querkräfte übertragen werden können. Der Antrieb 26 ist über eine Montage in einer Zwischenwand 27 lagefixiert am Gehäuseunterteil 4 angeordnet.

Der Antrieb 26 ist über eine Antriebsstange 37 und eine Kupplungsstange 38 wirktechnisch mit dem Kolben 15 kuppelbar. Sowohl die Antriebsstange 37 als auch die Kupplungsstange 38 sind Bestandteil der Infusionspumpe 1 und dauerhaft miteinander und mit dem Antriebsmotor 26 gekoppelt, also nicht dazu bestimmt, durch einen Anwender von diesem entkoppelt zu werden. Die Antriebsstange 37 ist mittels des Antriebsmotors 26 angetrieben und kann infolgedessen eine Hin-Her-Bewegung in Richtung der Längsachse L ausführen. An ihrem distalen Ende weist die Kupplungsstange 38 eine Gegenkuppelstruktur 40 auf, die passend zur Kuppelstruktur 34 des Kolbens 15 ausgebildet ist. Die Gegenkuppelstruktur 40 besteht hier in einem Bereich verminderten Durchmessers mit einer ggf. umlaufenden Vertiefung 41 oder Nut 41, die für ein kuppelndes Eingreifen mit den Kuppelhaken 36 des Kolbens 15 geeignet und bestimmt ist.

Auf der Kupplungsstange 38 ist ein Sicherungselement 39 hier in Form einer Klemmhülse 39 relativ zur Kupplungsstange 38 in Richtung der Längsachse L zwischen einer distalen Klemmstellung und einer proximalen Freigabestellung positionierbar angeordnet. In der Klemmstellung (siehe Figur 4) übergreift die Klemmhülse 39 mit ihren distalen Endabschnitt die in die Vertiefung 41 eingreifenden Kuppelhaken 36, so dass sich diese nicht durch eine Verformung nach radial außen aus der Vertiefung lösen können und die Verbindung von Kuppelstruktur 34 und Gegenkuppelstruktur 40 gesichert ist. In der Freigabestellung (Figur 5) gibt der distale Endabschnitt der Klemmhülse 39 die Kuppelhaken 36 frei, so dass sich diese in radialer Richtung nach außen verformen und aus der Vertiefung 41 lösen können, wenn ein Anwender das Pumpmodul 2 am Griffelement 25 ergreift und in distaler Richtung aus der Aufnahme 30 herauszieht. Die Klemmhülse 39 ist mittels einer Druckfeder 42 in die distale Richtung, also in ihre Klemmstellung, vorgespannt.

Die Infusionspumpe 2 weist außerdem eine Steuerkulisse 43 auf. Diese ist in einem Rahmen 46 des Gehäuseunterteils 4 in einer Richtung quer zur Längsrichtung L zwischen einer Entkopplungsstellung / Sperrstellung (Figur 2) und einer Freigabestellung / Pumpstellung (Figur 3) positionierbar aufgenommen. Die Steuerkulisse 43 ist mit einem Kniehebelmechanismus 47 gekoppelt, der im Wesentlichen aus einem ersten Hebelarm 48 und einem mit diesem über ein freies Gelenk 49 verbundenen zweiten Hebelarm 50 besteht. Der erste Hebelarm 48 ist im Wesentlichen L-förmig ausgebildet und mittels eines zum Gehäuseunterteil 4 fixen Drehgelenks 51 schwenkbar am Gehäuseunterteil 4 angeordnet. Er ist mit seinem dem freien Gelenk 49 gegenüberliegenden Ende mit einer mit dem Deckelelement 6 zusammenwirkenden Betätigungsstange 53 gekoppelt. Der zweite Hebelarm 50 ist mit seinem dem freien Gelenk 49 gegenüberliegenden Ende drehbar mit der Steuerkulisse 43 gekoppelt.

Die Steuerkulisse 43 weist eine gehäuseunterteilseitige Unterwand 54, eine dieser gegenüberliegende gehäuseoberteilseitige Oberwand 55 und zwei die Oberwand 55 und die Unterwand 54 verbindende Seitenwände 56 auf. Der zweite Hebelarm 50 ist mit der einen Seitenwand 56 gekoppelt, während die andere Seitenwand 56 mit einer am Rahmen 46 abgestützten Druckfeder 57 gekoppelt ist, die die Steuerkulisse 43 in Richtung des zweiten Hebelarms 50 (in den Figuren 1, 2 und 3 nach rechts, in den Figuren 4 und 5 aus der Zeichenebene heraus) vorspannt. In der Unterwand 54 (und identisch in der Oberwand 55) ist eine Steuernut 58 ausgebildet. Diese weist einen in Längsrichtung L verlaufenden Freigabeabschnitt 59 (siehe in Figur 2) und einen zunächst geneigt und dann quer zur Längsrichtung L verlaufenen Sperrabschnitt 60 (siehe in Figur 3) auf.

Die Klemmhülse 39 weist zwei einander diametral gegenüberliegende Steuernocken 61 auf, die in die Steuernuten 58 der Oberwand 55 und der Unterwand 54 eingreifen.

Aus den Figuren 2 bis 5 ergibt sich, dass das Deckelelement 6 in Form der Verschlussklappe 6 zwischen einer geöffneten Stellung (Figur 3) und einer geschlossenen Stellung (Figuren 2, 4 und 5) schwenkbar mittels eines Scharniergelenks 62 am Gehäuseunterteil 4 angeordnet ist. Es ist mit einem Positionierungszapfen 63 versehen, der mit dem zweiten Schlauchabschnitt 13 zusammenwirkt und sicherstellt, dass dieser bei geschlossener Verschlussklappe 6 bestimmungsgemäß an einem Luftsensor 44 platziert ist.

Zum Verwenden der Pumpe 1 öffnet ein Anwender zuerst die Verschlussklappe 6 der Pumpe 1, zum Beispiel indem er einen Spezialschlüssel seitlich in eine dafür vorgesehene Bohrung einführt und die Klappe 6 damit entriegelt. Wenn die Frontklappe 6 geöffnet ist, befindet sich die Steuerkulisse 43 in der Entkopplungs- oder Sperrstellung und die Kupplungsstange 38 im ausgefahrenen Zustand. Dadurch wurde die Betätigungsstange 53 mittels der beiden Hebelarme 48, 50 nach außen (distal) geschoben. Nachdem die Infusionsleitung einschließlich der beiden Schlauchabschnitte 12, 13 luftblasenfrei befüllt ist, wird der zweite Schlauchabschnitt 13 mittels einer Schiebeklemme 45 fluiddicht verschlossen. Danach schiebt der Anwender das Pumpmodul 2 in die dazu vorgesehene Aufnahme 30 der Pumpe 1, die in dem in Figur 2 gezeigten Zustand mit in der Entkopplungs- oder Sperrstellung befindlicher Steuerkulisse 43 (in Figur 2 nach rechts verschoben) und in der Freigabestellung befindlicher Klemmhülse 39 (in die proximale Richtung verschoben) ist, bis die Kuppelstruktur 34 mit der Gegenkuppelstruktur 40, d.h. die Kuppelhaken 36 in die Vertiefung 41 vorzugsweise hör- und/oder fühlbar einrastet bzw. einrasten und die Kolben-Zylinder-Einheit 11 mit dem Antrieb 26 verbunden ist.

Nachfolgend wird die Verschlussklappe 6 verschlossen. Dabei wirkt die Verschlussklappe 6 mit der Betätigungsstange 53 zusammen und verschiebt diese in Richtung proximal. Infolgedessen führt der erste Hebelarm 48 eine Schwenkbewegung (in den Figuren 2 und 3 entgegen dem Uhrzeigersinn) um das fixe Drehgelenk 51 aus, wodurch die Steuerkulisse 43 mittels des zweiten Hebelarms 50 entgegen der Vorspannung der Druckfeder 57 in dem Rahmen 46 bewegt wird (aus der in Figur 2 gezeigten Sperrstellung nach links in die in Figur 3 gezeigte Freigabe- oder Pumpstellung). Dabei wird die Klemmhülse 39 mittels der in die Steuernut 58 eingreifenden Steuernocken 61 in Richtung distal in ihre Klemmstellung bewegt, in der die Kuppelstruktur 34 und die Gegenkuppelstruktur 40 zueinander gesichert sind. Die an der Klemmhülse 39 befindliche Druckfeder 42 sorgt für eine sichere Verbindung während des Betriebs.

Ein im Gehäuseoberteil 5 befindlicher Verschlussmechanismus sorgt für eine sichere Arretierung der Klappe 6 in der geschlossenen Stellung. Gleichzeitig wird der zweite Schlauchabschnitt 13 bestimmungsgemäß zum Luftsensor platziert, zum Beispiel an diesen gedrückt. Ein Erkennen und entsprechendes Kommunizieren von eventuell vorhandenen oder eingebrachten Luftblasen bei einer Infusion ist damit sichergestellt.

Jetzt gibt der Anwender die gewünschte Förderrate über die Anzeigeelemente 8 und die Bedienungselemente 9 ein. Deren Darstellung erfolgt über das Display 10, das nach einer Ausführungsform als Touch-Screen ausgebildet sein kann, so dass eine Eingabe auch über das Display 10 erfolgen kann (eine Bedienung über eine App im Handy oder Tablet ist außerdem möglich und im Rahmen der Erfindung). Unmittelbar nach Freigabe startet die Pumpe 1. Sobald der Anwender die Infusion gestartet hat, bewegt sich die Antriebsstange 37 des Linearmotors 46 mit der angekoppelten Kuppelstange 38 inclusive Klemmhülse 39 und Kolben 15 in dem Freigabeabschnitt 59 der Steuernut 58 der Steuerkulisse 43 hin und her, und pumpt dabei Infusionslösung in Richtung Patient.

Nach Beendigung der Infusion wird die Pumpe 1 abgeschaltet. Die Antriebsstange 37 des Linearmotors 46 fährt die Kuppelstange 38 mit dem Kolben 15 zurück in die Ausgangsstellung. Sobald der Anwender das Deckelelement 6 öffnet, drückt die Druckfeder 57 die Steuerkulisse 43 in Richtung der Sperrstellung (in den Figuren 2 und 3 nach rechts) zurück. Dabei wird die Betätigungsstange 53 über den Kniehebelmechanismus 47 wieder nach außen (distal) bewegt. Durch die Bewegung der Steuerkulisse 43 zurück in die Sperrstellung wird die Klemmhülse 39 infolge des Eingriffs der Steuernocken 61 in die Steuernut 58 in Richtung proximal relativ zur Kuppelstruktur 34 und Gegenkuppelstruktur 40 positioniert und gibt diese frei, so dass der Kolben 15 von der Kuppelstange 38 unter Ausfedern der Kuppelhaken 36 gelöst werden kann. Nun kann der Anwender das Pumpmodul 2 aus der Aufnahme 30 der Infusionspumpe 1 entnehmen.

Im Falle eines Notfalls oder sollte die Infusionspumpe 1 einen technischen Defekt haben, kann man den Kolben 15 über ein Notentriegelung mit Hilfe des Betätigungselements 16 entriegeln. Hierbei (siehe Figuren 4 und 5) wird das Betätigungselement 16 manuell in Richtung proximal geschoben (siehe auch Pfeil auf dem Betätigungselement 16 in Figur 1). Ein Mitnehmernocken 64, der mit dem Steuernocken 61 der Klemmhülse 39 in Eingriff steht, schiebt diesen mit der Klemmhülse 39 dabei nach hinten (in Richtung proximal), so dass die Kuppelstruktur 34 und die Gegenkuppelstruktur 40 freigegeben sind und der Kolben 15 von der Kupplungsstange 38 getrennt werden kann. Damit die Notentriegelung nicht in der Freigabe- oder Pumpstellung (dargestellt in Figur 3), sondern nur in der Entkopplungs- oder Sperrstellung (dargestellt in Figur 2) funktioniert, befindet sich (siehe insbesondere in Figur 5) auf der Steuerkulisse 43 ein Steg 65 oder Nocken 65, der bei in der Freigabe- oder Pumpstellung befindlicher Steuerkulisse 43 mit dem Mitnehmernocken 64 zusammenwirkt und verhindert, dass ein Anwender die Notentriegelung versehentlich während der Infusion auslösen kann. Alternativ kann man die Steuerkulisse 43 über einen weiteren Linearmotor steuern.

### Bezugszeichenliste

- 1: Infusionspumpe
- 2: Pumpmodul
- 3: Gehäuse
- 4: Gehäuseunterteil
- 5: Gehäusedeckel
- 6: Deckelelement, Verschlussklappe
- 7: Gehäuseboden
- 8: Anzeigeelemente
- 9: Bedienungselemente
- 10: Anzeigedisplay
- 11: Kolben-Zylinder-Einheit
- 12: erster Schlauchabschnitt
- 13: zweiter Schlauchabschnitt
- 14: Zylinder
- 15: Kolben
- 16: Betätigungselement, Schieberiegel
- 17: Bodenwand
- 18: Umfangswand
- 19: Deckelwand
- 20: Dichtung
- 21: Fluidraum / Dosierraum
- 22: Einlaufbohrung
- 23: Auslaufbohrung
- 24: Zylinderkopf
- 25: Griffelement
- 26: Antrieb, Linearmotor
- 27: Zwischenwand
- 28: Steuereinheit
- 29: Zwischenwand
- 30: Aufnahme
- 31: Fluidkanal
- 32: Bodenwand
- 33: Seitenwand
- 34: Kuppelstruktur
- 35: Sackloch
- 36: Kuppelhaken
- 37: Antriebsstange
- 38: Kupplungsstange
- 39: Sicherungselement, Klemmhülse
- 40: Gegenkuppelstruktur
- 41: Vertiefung, Nut
- 42: Druckfeder
- 43: Steuerkulisse
- 44: Luftsensor
- 45: Schiebeklemme
- 46: Rahmen
- 47: Kniehebelmechanismus
- 48: erster Hebelarm
- 49: freies Gelenk
- 50: zweiter Hebelarm
- 51: Drehgelenk
- 52: Ringwulst
- 53: Betätigungsstange
- 54: Unterwand
- 55: Oberwand
- 56: Seitenwände
- 57: Druckfeder
- 58: Steuernut
- 59: Freigabeabschnitt
- 60: Sperrabschnitt
- 61: Steuernocken
- 62: Scharniergelenk
- 63: Positionierungszapfen
- 64: Mitnehmernocken
- 65: Steg, Nocken

- L: Längsrichtung, Achsrichtung

## Patentansprüche

1. Infusionssystem mit einer Infusionspumpe (1) und einem hierzu separaten, als Einwegprodukt vorgesehenen und damit austauschbaren Pumpmodul (2), das in einer Aufnahme (30) der Infusionspumpe (1) anordbar oder angeordnet ist und eine mit einem Antriebsmechanismus (26) der Infusionspumpe (1) wirkverbindbare Kolben-Zylinder-Einheit (11) mit einem in einem Zylinder (14) translatorisch bewegbaren Kolben (15) aufweist, der mittels einer mit ihm wirkverbundenen Koppelstruktur (34) und mittels einer zum verbindenden Eingreifen mit der Koppelstruktur (34) ausgebildeten und mit dem Antriebsmechanismus (26) wirkverbundenen Gegenkoppelstruktur (40) mit dem Antriebsmechanismus (26) koppelbar ist, **dadurch gekennzeichnet, dass** die Infusionspumpe (1) eine Steuerkulisse (43) aufweist, wobei die Steuerkulisse (43) derart mit dem Pumpmodul (2) wirkverbunden oder wirkverbindbar ist, dass sie bei bestimmungsgemäß in der Aufnahme (30) angeordnetem Pumpmodul (2) in eine Freigabestellung zwangspositioniert ist, in der eine Pumpaktion des Kolbens (15) durch die Steuerkulisse (43) freigegeben wird, und dass sie bei nicht bestimmungsgemäß in der Aufnahme (30) angeordnetem Pumpmodul (2) in eine Sperrstellung zwangspositioniert ist, in der eine Pumpaktion des Kolbens (15) durch die Steuerkulisse (43) gesperrt wird.

2. Infusionssystem nach Anspruch 1, **dadurch gekennzeichnet, dass** das Infusionssystem ferner ein Sicherungselement (39) aufweist, das dafür ausgebildet ist, in einer Sicherungsstellung die Koppelstruktur (34) und die Gegenkoppelstruktur (40) im gekoppelten Zustand miteinander zu verriegeln.

3. Infusionssystem nach Anspruch 1 oder 2, **dadurch gekennzeichnet, dass** die Infusionspumpe (1) ein Deckelelement (6) aufweist, insbesondere in Form einer Deckelklappe (6), das in eine die Aufnahme (30) für das Pumpmodul (2) verschließende Schließstellung positionierbar ist, wobei das Deckelelement (6), die Aufnahme (30) und das Pumpmodul (2) derart aufeinander abgestimmt und ausgebildet sind, dass das Deckelelement (6) bei nicht bestimmungsgemäß in der Aufnahme (30) angeordnetem Pumpmodul (2) nicht in der die Aufnahme (30) verschließende Schließstellung positionierbar ist.

4. Infusionssystem nach Anspruch 3, **dadurch gekennzeichnet, dass** das Deckelelement (6) mit der Steuerkulisse (43) wirkverbunden ist, derart, dass die Steuerkulisse (43) bei in der Schließstellung befindlichem Deckelelement (6) in die Freigabestellung zwangspositioniert ist, und bei nicht in der Schließstellung befindlichem Deckelelement (6) in die Sperrstellung zwangspositioniert ist.

5. Infusionssystem nach einem der Ansprüche 2 bis 4, **dadurch gekennzeichnet, dass** das Sicherungselement (39) mit der Steuerkulisse (43) wirkverbunden ist, derart, dass das Sicherungselement (39) die Koppelstruktur (34) und die Gegenkoppelstruktur (40) bei in der Freigabestellung befindlicher Steuerkulisse (43) fixiert und das Sicherungselement (39) die Koppelstruktur (34) und die Gegenkoppelstruktur (40) bei in der Sperrstellung befindlicher Steuerkulisse (43) freigibt.

6. Infusionssystem nach einem der Ansprüche 3 bis 5, **dadurch gekennzeichnet, dass** das Infusionssystem, insbesondere die Infusionspumpe (1), einen Kniehebelmechanismus (47) aufweist, der einerseits mit dem Pumpmodul (2) wirkverbunden ist, insbesondere über eine Ankopplung an das Deckelelement (6), und andererseits mit der Steuerkulisse (43) wirkverbunden ist, insbesondere an der Steuerkulisse (43) angelenkt ist.

7. Infusionssystem nach einem der vorstehenden Ansprüche, **dadurch gekennzeichnet, dass** die Steuerkulisse (43) in die Sperrstellung vorgespannt ist, insbesondere mittels eines Federelements (57) wie einer Biegefeder, Druckfeder oder Zugfeder.

8. Infusionssystem nach einem der Ansprüche 2 bis 7, **dadurch gekennzeichnet, dass** das Sicherungselement (39) als hülsenförmiger Hohlkörper ausgebildet ist, dessen Längsachse parallel zur, insbesondere kongruent mit der, Kolbenachse ausgerichtet ist, und dass das Sicherungselement (39) relativ zur Koppelstruktur (34) und Gegenkoppelstruktur (40) in Richtung der Kolbenachse zwischen der Sicherungsstellung und einer Freigabestellung positionierbar ist.

9. Infusionssystem nach einem der Ansprüche 2 bis 8, **dadurch gekennzeichnet, dass** das Sicherungselement (39) in die Sicherungsstellung vorgespannt ist, insbesondere mittels eines Federelements (42) wie einer Biegefeder, Druckfeder oder Zugfeder.

10. Infusionssystem nach einem der Ansprüche 2 bis 9, **dadurch gekennzeichnet, dass** das Infusionssystem eine Notentriegelung mit einem mit dem Sicherungselement (39) gekoppelten Betätigungselement (16) aufweist, um das Sicherungselement (39) manuell zu betätigen und aus der Sicherungsstellung in eine Freigabestellung zu bringen.

11. Infusionssystem nach Anspruch 10, **dadurch gekennzeichnet, dass** das Betätigungselement (16) mit der Steuerkulisse (43) gekoppelt ist, derart, dass es bei in der Freigabestellung befindlicher Steuerkulisse (43) blockiert und nicht zu betätigen ist, um das Sicherungselement (39) aus der Sicherungsstellung in eine Freigabestellung zu bringen.

## Claims

1. An infusion system comprising an infusion pump (1) and a pump module (2) being separate therefrom and provided as a disposable product and thus being replaceable and which can be or is arranged in a mount (30) of the infusion pump (1) and includes a plunger-barrel unit (11) operatively connectable to a drive mechanism (26) of the infusion pump (1) comprising a plunger (15) translationally movable within a barrel (14), the plunger being adapted to be coupled to the drive mechanism (26) by means of a coupling structure (34) operatively connected to the plunger and by means of a counter-coupling structure (40) configured for connecting engagement in the coupling structure (34) and being operatively connected to the drive mechanism (26), **characterized in that** the infusion pump (1) has a control link (43), wherein the control link (43) is operatively connected or operatively connectable to the pump module (2) so that, when the pump module (2) is arranged in the mount (30) as intended, the control link is positioned by force in a release position in which pumping action of the plunger (15) gets released by the control link (43), and that, when the pump module (2) is not arranged in the mount (30) as intended, the control link is positioned by force in a blocking position in which pumping action of the plunger (15) gets locked by the control link (43).

2. The infusion system according to claim 1, **characterized in that** the infusion system further includes a securing member (39) which is designed for locking the coupling structure (34) and the counter-coupling structure (40) in the coupled state to each other in a securing position.

3. The infusion system according to claim 1 or 2, **characterized in that** the infusion pump (1) includes a cover member (6), especially in the form of a cover flap (6), which can be positioned in a closing position closing the mount (30) for the pump module (2), wherein the cover member (6), the mount (30) and the pump module (2) are adapted to each other and designed so that, when the pump module (2) is not arranged in the mount (30) as intended, the cover member (6) cannot be positioned in the closing position closing the mount (30).

4. The infusion system according to claim 3, **characterized in that** the cover member (6) is operatively connected to the control link (43) in such a way that the control link (43) is positioned by force into the release position when the cover member (6) is provided in the closing position and is positioned by force into the blocking position when the cover member (6) is not provided in the closing position.

5. The infusion system according to one of the claims 2 to 4, **characterized in that** the securing member (39) is operatively connected to the control link (43) in such a way that the securing member (39) fixes the coupling structure (34) and the counter-coupling structure (40) when the control link (43) is provided in the release position, and the securing member (39) releases the coupling structure (34) and the counter-coupling structure (40) when the control link (43) is provided in the blocking position.

6. The infusion system according to one of the claims 3 to 5, **characterized in that** the infusion system, especially the infusion pump (1), includes a toggle lever mechanism (47) which, on the one hand, is operatively connected to the pump module (2), especially by coupling to the cover member (6), and, on the other hand, is operatively connected to the control link (43), especially articulated to the control link (43).

7. The infusion system according to one of the preceding claims, **characterized in that** the control link (43) is prestressed to the blocking position, especially by means of a spring element (57) such as a flexural spring, compression spring or tension spring.

8. The infusion system according to one of the claims 2 to 7, **characterized in that** the securing member (39) is a sleeve-shaped hollow body, the longitudinal axis of which is oriented in parallel to, especially congruent to the plunger axis, and **in that** the securing member (39) can be positioned relative to the coupling structure (34) and the counter-coupling structure (40) in the direction of the plunger axis between the securing position and a release position.

9. The infusion system according to one of the claims 2 to 8, **characterized in that** the securing member (39) is biased to the securing position, especially by means of a spring element (42) such as a flexural spring, compression spring or tension spring.

10. The infusion system according to one of the claims 2 to 9, **characterized in that** the infusion system includes an emergency release comprising an actuating element (16) coupled to the securing member (39) so as to manually actuate the securing member (39) and to move the same from the securing position to a release position.

11. The infusion system according to claim 10, **characterized in that** the actuating element (16) is coupled to the control link (43) so that, when the control link (43) is provided in the release position, it will block and cannot be actuated for moving the securing member (39) from the securing position to a release position.

## Revendications

1. Système de perfusion avec une pompe à perfusion (1) et un module de pompe (2) séparé de celle-ci prévu en tant que produit à usage unique et échangeable avec celle-ci, qui peut être disposé ou est disposé dans un logement (30) de la pompe à perfusion (1) et présente une unité piston-cylindre (11) qui peut être reliée en fonctionnement avec un mécanisme d'entraînement (26) de la pompe à perfusion (1) avec un piston (15) mobile en translation dans un cylindre (14), lequel piston peut être couplé au mécanisme d'entraînement (26) au moyen d'une structure de couplage (34) reliée fonctionnement à celui-ci et au moyen d'une structure de contre-couplage (40) réalisée pour un enclenchement de liaison avec la structure de couplage (34) et reliée fonctionnellement au mécanisme d'entraînement (26), **caractérisé en ce que**
la pompe à perfusion (1) présente une glissière de commande (43), dans lequel la glissière de commande (43) est reliée en fonctionnement ou peut être reliée en fonctionnement avec le module de pompe (2) de telle sorte qu'elle est positionnée de force dans une position de libération lorsque le module de pompe (2) est disposé dans le logement (30) conformément à l'usage prévu, position dans laquelle une action de pompage du piston (15) est libérée par le biais de la glissière de commande (43) et **en ce que** lorsque le module de pompe (2) n'est pas disposé dans le logement (30) conformément à l'usage prévu elle est positionnée de force dans une position de verrouillage dans laquelle une action de pompage du piston (15) est bloquée par le biais de la glissière de commande (43).

2. Système de perfusion selon la revendication 1, **caractérisé en ce que** le système de perfusion présente en outre un élément de sécurisation (39) qui est réalisé pour verrouiller ensemble la structure de couplage (34) et la structure de contre-couplage (40) à l'état couplé dans une position de sécurisation.

3. Système de perfusion selon la revendication 1 ou 2, **caractérisé en ce que** la pompe à perfusion (1) présente un élément de couvercle (6), en particulier sous la forme d'un rabat de couvercle (6) qui peut être positionné dans une position de fermeture fermant le logement (30) pour le module de pompe (2), dans lequel l'élément de couvercle (6), le logement (30) et le module de pompe (2) sont coordonnés et réalisés de telle sorte que l'élément de couvercle (6), lorsque le module de pompage (2) n'est pas disposé dans le logement (30) conformément à l'usage prévu, ne puisse pas positionné dans la position de fermeture qui ferme le logement (30).

4. Système de perfusion selon la revendication 3, **caractérisé en ce que** l'élément de couvercle (6) est relié en fonctionnement avec la glissière de commande (43) de telle sorte que la glissière de commande (43) est positionnée de force dans la position de libération lorsque l'élément de couvercle (6) se trouve dans la position de fermeture et est positionnée de force dans la position de verrouillage lorsque l'élément de couvercle (6) ne se trouve pas dans la position de fermeture.

5. Système de perfusion selon l'une quelconque des revendications 2 à 4, **caractérisé en ce que** l'élément de sécurisation (39) est relié en fonctionnement avec la glissière de commande (43) de telle sorte que l'élément de sécurisation (39) fixe la structure de couplage (34) et la structure de contre-couplage (40) lorsque la glissière de commande (43) se trouve dans la position de libération, et l'élément de sécurisation (39) libère la structure de couplage (34) et la structure de contre-couplage (40) lorsque la glissière de commande (43) se trouve dans la position de verrouillage.

6. Système de perfusion selon l'une quelconque des revendications 3 à 5, **caractérisé en ce que** le système de perfusion, en particulier la pompe à perfusion (1), présente un mécanisme à genouillère (47) qui est relié en fonctionnement d'un côté avec le module de pompe (2), en particulier par le biais d'un couplage à l'élément de couvercle (6) et d'un autre côté avec la glissière de commande (43), en particulier est articulé au niveau de la glissière de commande (43).

7. Système de perfusion selon l'une quelconque des revendications précédentes, **caractérisé en ce que** la glissière de commande (43) est précontrainte dans la position de verrouillage, en particulier au moyen d'un élément à ressort (57) comme un ressort de flexion, un ressort de compression ou un ressort de traction.

8. Système de perfusion selon l'une quelconque des revendications 2 à 7, **caractérisé en ce que** l'élément de sécurisation (39) est réalisé en tant que corps creux en forme de douille, dont l'axe longitudinal est orienté parallèlement, en particulier de manière congruente, avec l'axe de piston et **en ce que** l'élément de sécurisation (39) peut être positionné par rapport à la structure de couplage (34) et à la structure de contre-couplage (40) en direction de l'axe de piston entre la position de sécurisation et une position de libération.

9. Système de perfusion selon l'une quelconque des revendications 2 à 8, **caractérisé en ce que** l'élément de sécurisation (39) est précontraint dans la position de sécurisation, en particulier au moyen d'un élément à ressort (42) comme un ressort de flexion, un ressort de compression ou un ressort de traction.

10. Système de perfusion selon l'une quelconque des revendications 2 à 9, **caractérisé en ce que** le système de perfusion présente un déverrouillage d'urgence avec un élément d'actionnement (16) couplé à l'élément de sécurisation (39) pour actionner manuellement l'élément de sécurisation (39) et le faire passer de la position de sécurisation à une position de libération.

11. Système de perfusion selon la revendication 10, **caractérisé en ce que** l'élément d'actionnement (16) est couplé avec la glissière de commande (43) de telle sorte qu'il est bloqué lorsque la glissière de commande (43) se trouve dans la position de libération pour faire passer l'élément de sécurisation (39) de la position de sécurisation à une position de libération.
